# EUROPEAN PATENT APPLICATION

(11) **EP 4 663 771 A1**
(43) Date of publication of application: **17.12.2025**
(21) Application number: 24382628.6
(22) Date of filing: 10.06.2024
(51) Int. Cl.: C12Q 1/6844, C12Q 1/6881

(54) **METHODS TO ENRICH POPULATION OF CELLS FROM AN HETEROGENEOUS SAMPLE**

(71) Applicant: Bioliquid Innovative Genetics, S.L, 08036 Barcelona (ES)
(72) Inventor: SANTAMARIA COSTA, Xavier, 08036 Barcelona (ES); VALLVÉ JUANICO, Julia, 08036 Barcelona (ES); CAMPOY MONCAYO, Irene, 08036 Barcelona (ES); LAO MULINARI, Joan Ramon, 08036 Barcelona (ES)
(74) Representative: Hoffmann Eitle

(57) **Abstract**

The present invention provides an in vitro method for separating a first population of cells from a second population of cells comprised in a sample, wherein the first population of cells is characterized in that they comprise at least one target nucleic acid molecule that is not present in the second population of cells.

## Description

### TECHNICAL FIELD

The present invention relates to the field of biotechnology. Particularly, the present invention relates to the field of cell separation or enrichment methods.

### BACKGROUND ART

There are multiple methods that can be used when performing cell isolation. Some of them include Immunomagnetic Cell Separation, Fluorescence-activated Cell Sorting, Density Gradient Centrifugation, Immunodensity Cell Separation, Sedimentation, Adhesion, Microfluidic Cell Separation, etc.

However, the state-of-the-art methods lose sensitivity especially when using heterogeneous samples having multiple population of cells. The loss of sensitivity is even more accentuated when the heterogeneous sample comprises two population of cells that are very similar and may only be differentiated by the differential expression of certain genes. This is, for example, a sample having blood isolated from a pregnant woman, where the blood comprises foetal erythroblasts and mothers' erythroblasts. In this case, isolating the foetal erythroblasts from the mother's blood is challenging and the method described so far do not provide solutions that can be applied at a point of care.

Additionally, many of said method imply subjecting the sample and the cells comprised therein to reagents and/or conditions that result in the loss of cell's integrity. This is detrimental because in certain cases it is important to maintain viable cells after performing the cell separation method. Therefore, there is a need for efficient and sensitive cell separation methods that preserve cell's integrity and that are able to efficiently separate at least two population of cells that are very similar and mixed in a sample. The present invention aims at providing a solution for said needs.

### BRIEF DESCRIPTION OF DRAWINGS

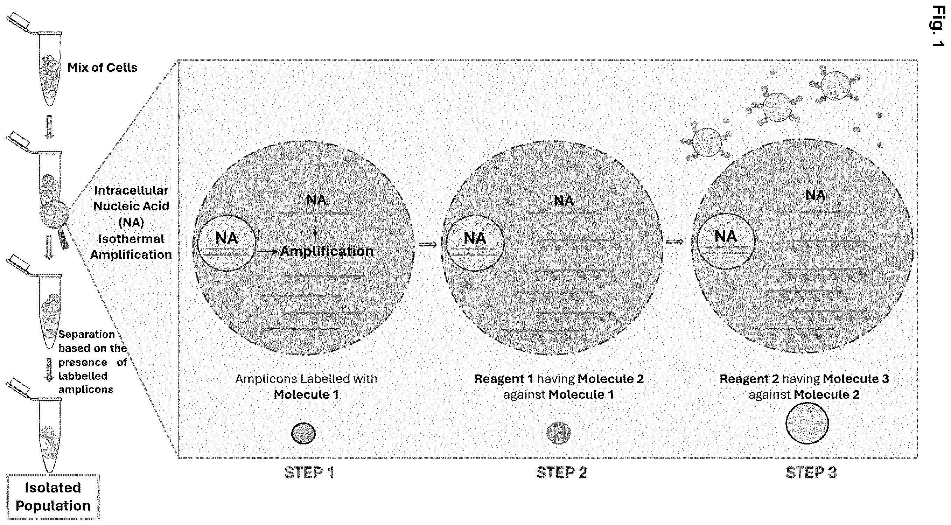

**Fig. 1****: Example of the method.** The figure shows the general process of cell isolation. Briefly, it consists in amplifying a specific acid nucleic inside the cell present in one population and not the other. The resulted amplicon is labelled with a molecule (Molecule 1). A second step labels the Molecule 1 with a specific Molecule 2 against this Molecule 1. The third step consists in washing the non-bound molecules 1 and 2 from inside the cell with a Molecule 3. After the process, the labelled cells can be isolated.

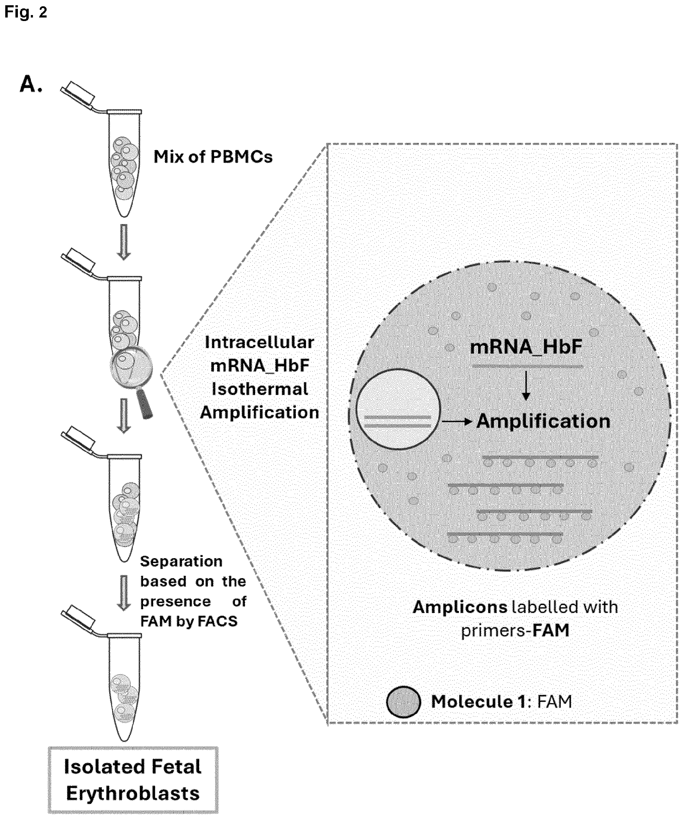

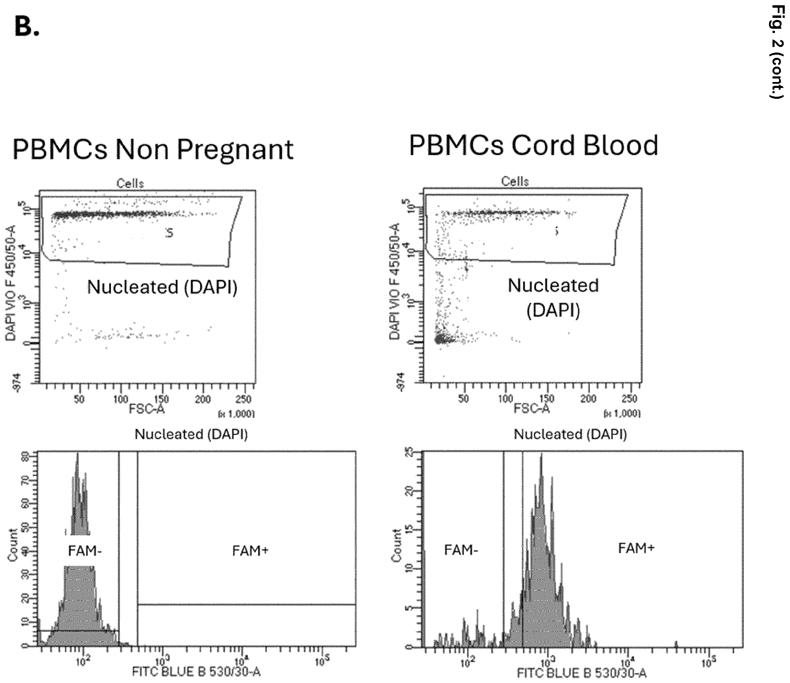

**Fig. 2****: Separation of fetal erythroblasts using primers labelled with FAM. A)** Process followed to isolate fetal erythroblasts. In this example, RT-LAMP was performed to amplify fetal hemoglobin (HbF) mRNA using primers that contain FAM and cells were detected and sorted by FACS. **B)** The left graphs show the cytometry dot plots for the negative control (PBMCs from a non-pregnant women) and the right graphs show the cytometry dot plots of PBMCs from umbilical cord blood. The top graphs show the percentage of nucleated cells (DAPI+) and the histograms show the positive and negative cells as FAM+ and FAM-, respectively.

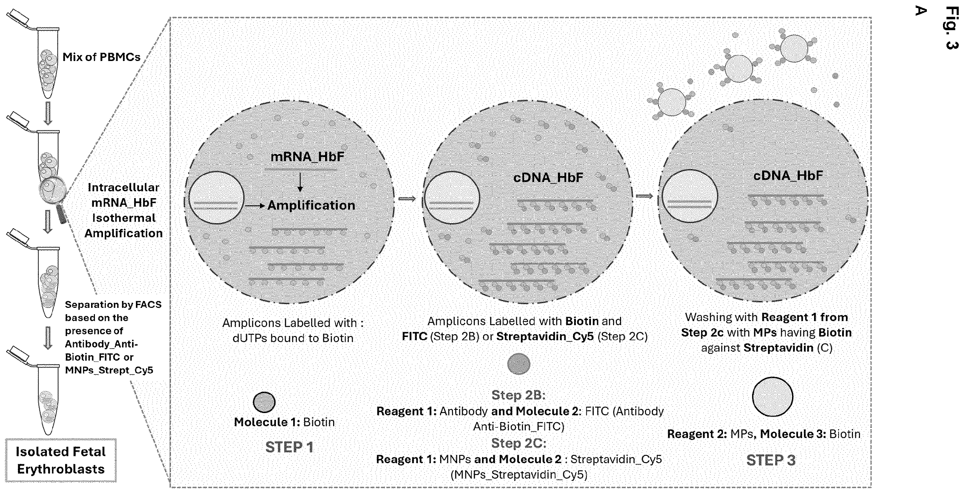

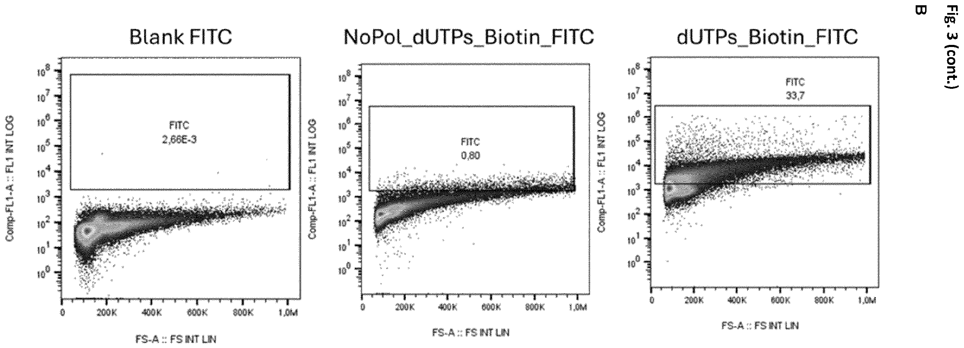

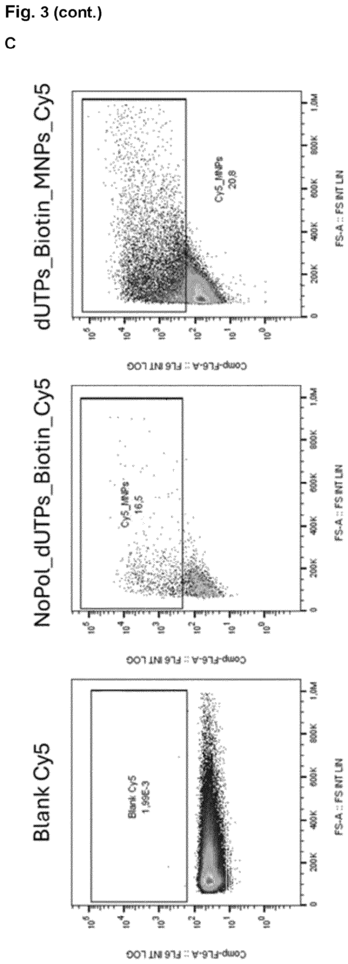

**Fig. 3****: Separation of fetal erythroblasts using dUTPs labelled with Biotin. A)** Explanation of the whole process carried out to separate fetal erythroblasts by the detection of cells that have been amplified by in-cell RT-LAMP and the amplicons contain a specific label, in this case, Biotin. Step 1 indicates the intracellular isothermal amplification, in this case RT-LAMP, Step 2 indicates two possibilities (Step 2b and 2c) of incubation with a first reagent (reagent 1) and molecule 2, and Step 3 is an optional step available for Step 2c, in which the wash is performed with a second reagent (reagent 2) and Molecule 3. **B)** Cytometry dot plots of cells from a sample of cord blood, which its cDNA was labelled with dUTPs bound to Biotin (Molecule 1) during an isothermal amplification using RT-LAMP and stained with an antibody against Biotin bound to FITC (Molecule 2). The three dot plots represent the samples with: 1) *Blank FITC*: no antibody_anti_biotin_FITC staining as a negative control for FITC signal; 2) *NoPol_dUTPs_Biotin_FITC*: antibody_anti_biotin_FITC staining in a sample where RT-LAMP was performed without the Polymerase required for the amplification, as a negative control for the LAMP; and 3) *dUTPs_Biotin_FITC*: antibody_anti_biotin_FITC staining in a sample in which RT-LAMP was performed. **C)** Cytometry dot plots of cells from a sample of cord blood, which its cDNA was labelled with dUTPs bound to Biotin (Molecule 1) during an isothermal amplification using RT-LAMP and labelled with magnetic nanoparticles (MNPs) bound to Streptavidin (against Biotin) and the fluorophore Cy5. The three dot plots represent the samples with: 1) *Blank Cy5:* no MNP_Cy5 labelling as a negative control for Cy5 signal; 2) *NoPol_dUTPs_Biotin_Cy5:* MNPs_Cy5 labelling in a sample where RT-LAMP was performed without the Polymerase required for the amplification, as a negative control for the LAMP; and 3) *dUTPs_Biotin_MNPs_Cy5:* MNPs_Cy5 in a sample in which RT-LAMP was performed.

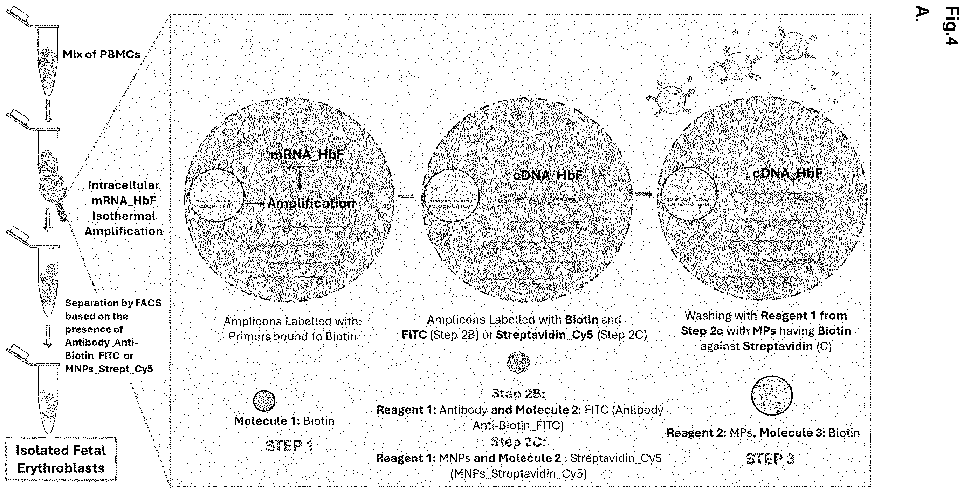

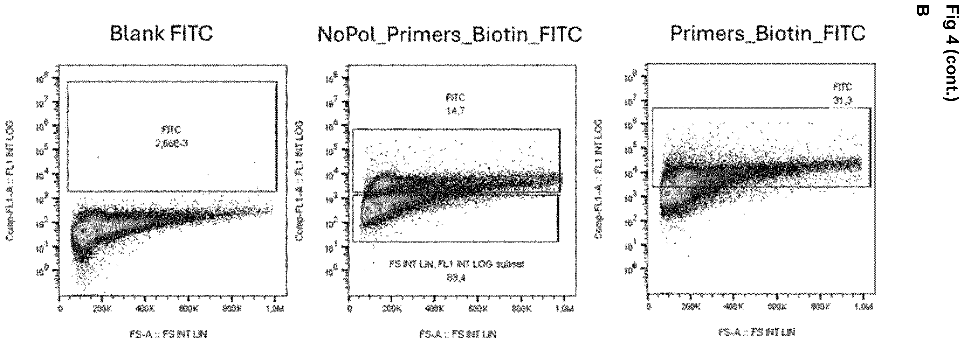

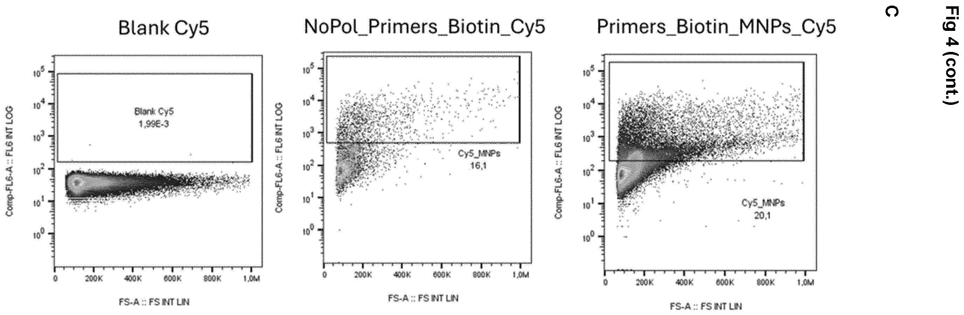

**Fig. 4****: Separation of fetal erythroblasts using primers labelled with Biotin. A)** Explanation of the whole process carried out to separate fetal erythroblasts by the detection of cells that have been amplified by in-cell RT-LAMP and the amplicons contain a specific label, in this case, Biotin. Step 1 indicates the intracellular isothermal amplification, in this case RT-LAMP, Step 2 indicates two possibilities (Step 2b and 2c) of incubation with a first reagent (reagent 1) and Molecule 2, and Step 3 is an optional step available for Step 2c, in which the wash is performed with a second reagent (reagent 2) and Molecule 3. **B)** Cytometry dot plots of cells from a sample of cord blood, which its cDNA was labelled with primers bound to Biotin (Molecule 1) during an isothermal amplification using RT-LAMP and stained with an antibody against Biotin bound to FITC (Molecule 2). The three dot plots represent the samples with: 1) *Blank FITC:* no antibody_anti_biotin_FITC staining as a negative control for FITC signal; 2) *NoPol_Primers_Biotin_FITC:* antibody_anti_biotin_FITC staining in a sample where RT-LAMP was performed without the Polymerase required for the amplification, as a negative control for the LAMP; and 3) *Primers_Biotin_FITC:* antibody_anti_biotin_FITC staining in a sample in which RT-LAMP was performed. **C)** Cytometry dot plots of cells from a sample of cord blood, which its cDNA was labelled with primers bound to Biotin (Molecule 1) during an isothermal amplification using RT-LAMP and labelled with magnetic nanoparticles (MNPs, reagent 1) bound to Streptavidin (against Biotin) and the fluorophore Cy5. The three dot plots represent the samples with: 1) *Blank Cy5:* no MNP_Cy5 labelling as a negative control for Cy5 signal; 2) *NoPol_Primers_Biotin_Cy5:* MNPs_Cy5 labelling in a sample where RT-LAMP was performed without the Polymerase required for the amplification, as a negative control for the LAMP; and 3) *Primers_Biotin_MNPs_Cy5:* MNPs_Cy5 in a sample in which RT-LAMP was performed.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

If the term "about" as used in connection with a numerical value throughout the specification and the claims denotes an interval of accuracy, familiar and acceptable to a person skilled in the art. For instance, the term "about" means the indicated value ± 1% of its value, or the term "about" means the indicated value ± 2% of its value, or the term "about" means the indicated value ± 5% of its value, the term "about" means the indicated value ± 10% of its value, or the term "about" means the indicated value ± 20% of its value, or the term "about" means the indicated value ± 30% of its value; preferably the term "about" means exactly the indicated value (± 0%).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "in-cell" as used herein refers to an assay that is performed inside the cells, i.e., a intracellular assay.. Thus, an in-cell amplification is an amplification carried out inside the cells. An in-cell reverse transcription is an reverse transcription that is carried out inside the cells (intracellularly).

### DESCRIPTION OF THE EMBODIMENTS

In **a first aspect,** the present provides a method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an isothermal amplification inside the cell of the target nucleic acid molecule comprised in the first population of cells and not in the second population of cells, wherein the isothermal amplification uses a first molecule that is incorporated in the resulting amplified nucleic acid molecule, thereby labelling said amplified nucleic acid molecule, and
b) detecting and separating the first population of cells from the second population of cells using a separation method that detects the presence of the labelled amplified target nucleic acid molecule in the first population of cells and separates the first population from the second population of cells in virtue of the presence of the labelled amplified nucleic acid molecule.

Therefore, provided herein are methods for enriching or separating or isolating a population of cells from a sample having at least two different population of cells.

Preferably, the method is an *in vitro* and/or *ex vivo* method.

### The sample

The sample may be any sample which contains at least two populations of cells, wherein one population of cells comprises a target nucleic acid molecule that is not present in the other population of cells. The sample includes both natural and synthetic samples, that is materials which occur naturally or preparations which have been made.

The sample is preferably a biological sample, more preferably an isolated biological sample. The sample, preferably isolated biological sample, is characterized by being heterogeneous, that is, by having at least two different population of cells, wherein the population of cells are differentiated by the presence of the target nucleic acid in one of the population of cells. The term "heterogeneous sample" refers to a sample that comprises more than one type of cells. Thus, the sample, preferably an isolated biological sample, comprises at least two population of cells, wherein a first population of cells comprises a target nucleic acid, and wherein the second population of cells do not comprise said target nucleic acid.

All biological and clinical samples are included, e.g. any cell or tissue sample of an organism, or any body-fluid or preparation derived therefrom, as well as samples such as cell cultures, cell preparations, cell lysates etc. The samples may be freshly prepared, or they may be prior-treated in any convenient way e.g. for storage.

The sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells. Exemplary biological samples include tissue samples (such as tissue sections and needle biopsies of a tissue); cell samples (e.g., cytological smears (such as Pap or blood smears) or samples of cells obtained by microdissection); samples of whole organisms (such as samples of yeasts or bacteria); or cell fractions, fragments or organelles (such as obtained by lysing cells and separating the components thereof by centrifugation or otherwise). Other examples of biological samples include blood, serum, urine, semen, fecal matter, cerebrospinal fluid, interstitial fluid, mucous, tears, sweat, pus, biopsied tissue (e.g., obtained by a surgical biopsy or needle biopsy), nipple aspirates, milk, vaginal fluid, saliva, swabs (such as buccal swabs), or any material containing biomolecules that is derived from a said biological sample. In some embodiments, the biological sample can be a body fluid, which can be fluid isolated from the body of an individual. For example, "body fluid" may include blood, plasma, serum, mucus, bile, saliva, nasopharyngeal swabs, urine, tears, perspiration, or washings from bodily cavities. Preferably, the sample is a biological sample, preferably blood.

As explained above, the first and second population of cells comprised in the heterogeneous sample are different in that at least the first population of cells comprises a target nucleic acid or a nucleotide sequence that is not present in the second population of cells. The first and second population of cells may be (i) fetal derived and maternal derived cells, (ii) cancer and non-cancer cells, (iii) pathogen and host cells, and more generally, (iv) mutated and wild-type cells.

Preferably, the sample is a mixed sample containing cells from the foetus (first population of cells) and from the mother (second population of cells). The sample may be maternal blood, maternal serum, maternal plasma, fetal cells, umbilical cord blood, chorionic villi, amniotic fluid, urine, saliva, lung lavage, cells or tissues. As used herein, the term "blood" encompasses whole blood or any fractions of blood, such as serum and plasma as conventionally defined, for example. Blood or fractions thereof. Blood also comprises buffy coats. Buffy coats are sometimes isolated by utilizing a ficoll gradient. Buffy coats can comprise white blood cells (e.g., leukocytes, T-cells, B-cells, platelets, and the like). In certain embodiments, the sample is an isolated buffy coat comprising maternal and fetal cells. Blood plasma refers to the fraction of whole blood resulting from centrifugation of blood treated with anticoagulants.

Preferably, the biological sample is a maternal blood sample, preferably maternal whole-blood sample. Preferably, the maternal blood is PMBCs obtained from the mother's blood. The term "maternal blood" as used herein refers to a blood sample or preparation from a pregnant woman or a woman being tested for possible pregnancy. The term encompasses whole blood or any fractions of blood, such as serum and plasma as conventionally defined, not only from the mother but also from the foetus. Preferably, the isolated biological sample is a whole blood sample obtained from a pregnant subject, preferably a pregnant woman, and the first population of cells is from the foetus, and the second population of cells is from the pregnant woman carrying said foetus. Preferably, the first population of cells are foetal blood cells, and the second population of cells are maternal blood cells. Preferably, the first population of cells are fetal erythroblasts, and the second population of cells are maternal erythroblasts.

Many laboratories around the world have been attempting for over a decade to develop non-invasive (i.e. venupuncture only) methods to isolate and analyse foetal cells. An obvious advantage is that definitive results can be obtained using molecular techniques such as fluorescence in-situ hybridization (FISH) and quantitative fluorescent polymerase chain reaction (QF-PCR) on recovered foetal cells.

The presence of foetal cells in maternal blood provides potentially the best possible source of cells for non-invasive prenatal diagnosis. However foetal cells are present at very low numbers, and their isolation is not a trivial task, with only 1 or 2 foetal cells being present per 10 ml maternal blood.

Therefore, in this preferred embodiment, the method is aimed at separating the first population of cells (preferably foetal cells), from the second population of cells (preferably mother's cells), based on the presence of a target nucleic acid molecule in the first population of cells (preferably foetal cells). Preferably, the target nucleic acid molecule is an mRNA molecule present in the foetal cells (i.e., in the first population of cells), but not present in the mother's cells (i.e., the second population of cells). Preferably, the target mRNA molecule is foetal hemoglobin mRNA or any mRNA molecule that is expressed in foetal cells but not in maternal cells. The use of the method of the present invention using blood of pregnant females to separate foetal cells facilitate prenatal diagnosis without invading foetal space. Therefore, the method of the present invention may be a method for enriching foetal cells obtained from a maternal sample.

The biological sample may be obtained from a subject in need of the analysis. A "subject" may be a human (i.e., male or female of any age group, for example, pediatric subject (e.g., infant, child, or adolescent) or adult subject (e.g., young adult, middle-aged adult, or senior adult). Alternatively, the subject may be a non-human animal. In certain embodiments, the non-human animal is a mammal (e.g., primate, for example, cynomolgus monkey or rhesus monkey), commercially relevant mammal (e.g., cattle, pig, horse, sheep, goat, cat, or dog), or bird (e.g., commercially relevant bird, such as chicken, duck, goose, or turkey). In other examples, the non-human animal is a fish, reptile, or amphibian. The non-human animal may be a transgenic animal or genetically engineered animal. In some examples, the subject may also be a plant.

For prenatal applications of the methods described herein, the sample may be collected from a female at a gestational age suitable for testing, or from a female who is being tested for possible pregnancy. Suitable gestational age may vary depending on the prenatal test being performed. In certain embodiments, a pregnant female subject sometimes is in the first trimester of pregnancy, at times in the second trimester of pregnancy, or sometimes in the third trimester of pregnancy. In certain embodiments, a fluid or tissue is collected from a pregnant female between about 1 to about 45 weeks of fetal gestation (e.g., at 1-4, 4-8, 8-12, 12-16, 16-20, 20-24, 24-28, 28- 32, 32-36, 36-40 or 40-44 weeks of fetal gestation), and sometimes between about 5 to about 28 weeks of fetal gestation (e.g., at 6, 7, 8, 9,10, 1 1 , 12, 13, 14, 15, 16, 17, 18, 19, 20, 21 , 22, 23, 24, 25, 26 or 27 weeks of fetal gestation). In certain embodiments a fluid or tissue sample is collected from a pregnant female during or just after (e.g., 0 to 72 hours after) giving birth (e.g., vaginal or non-vaginal birth (e.g., surgical delivery), or miscarriage).

In another embodiment, the biological sample may be a biopsy from a subject, preferably a human subject. Preferably, the first population of cells are cancerous cells, and the second population of cells are not cancerous cells, so that the method is performed to identify and separate the cancerous cells based on the present of a target nucleic acid that is not present in the non-cancerous cells. Thus, it is obvious to the skilled person that any sample can be used, as long as it is characterized by having at least two population of cells, wherein the first population of cells comprises a target nucleic acid molecule that is not present in the second population of cells.

### The target nucleic acid molecule

The target nucleic acid molecule is a polynucleotide or a nucleotide sequence that is present inside the first population of cells, but not present in the second population of cells. Thus, the target nucleic acid molecule is used as a differential feature between at least two populations of cells comprised in the sample. The target nucleic acid molecule may be any nucleotide sequence of interest that wants to be detected, analysed and/or amplified. The target nucleic acid molecule may include DNA (e.g. genomic DNA, mitochondrial DNA, plasMid DNA, viral DNA etc.) and RNA (e.g. mRNA, microRNA, rRNA, snRNA, viral RNA etc.), and synthetic and/or modified nucleic acid molecules, (e.g. including nucleic acid domains comprising or consisting of synthetic or modified nucleotides such as LNA, PNA, DNA/RNA hybrids, morpholino etc.), or fragments thereof. The target nucleic acid molecule may be a natural (i.e., naturally occurring nucleic acid), or an artificial nucleic acid. Thus, the target nucleic acid molecule may be made up of ribonucleotides and/or deoxyribonucleotides as well as synthetic nucleotides that are capable of participating in Watson-Crick type or analogous base pair interactions. The target nucleic acid molecule may be or may comprise, e.g. LNA, PNA or any other derivative containing a non-nucleotide backbone.

The target nucleic acid molecule may also be coding or non-coding DNA, for example genomic DNA or a sub-fraction thereof, or may be derived from genomic DNA, e.g. a copy or amplicon thereof, or it may be cDNA or a sub-fraction thereof, or an amplicon or copy thereof etc.

As noted above, the target nucleic acid molecule may be a RNA molecule. The target nucleic acid molecule may, for example, be an RNA molecule in a pool of RNA or other nucleic acid molecules or nucleotide sequences, for example genomic nucleic acids, whether human or from any source, from a transcriptome, or any other nucleic acid (e.g. organelle nucleic acids, i.e. mitochondrial or plastid nucleic acids), whether naturally occurring or synthetic. The target RNA may thus be or may be derived from coding (i.e. pre-mRNA or mRNA) or non-coding RNA sequences (such as tRNA, rRNA, snoRNA, miRNA, siRNA, snRNA, exRNA, piRNA and long ncRNA). Preferably, the target nucleic acid molecule is an mRNA molecule derived from a gene that is expressed in the first population of cells, but not expressed in the second population of cells.

The target nucleic acid molecule also may include, as equivalents, derivatives, variants and analogs of RNA or DNA synthesized from nucleotide analogs, single-stranded ("sense" or "antisense", "plus" strand or "minus" strand, "forward" reading frame or "reverse" reading frame) and double-stranded polynucleotides. Deoxyribonucleotides include deoxyadenosine, deoxycytidine, deoxyguanosine and deoxythymidine. For RNA, the base cytosine is replaced with uracil.

In an embodiment, the method of the first aspect is performed without disrupting or altering or breaking the cell's membrane of the first population of cells. Thus, all the reagents used in the present invention are capable of entering in the cells, that is, have a size smaller than the average cell's membrane pore (which is about 50nm), except the reagent called herein "the second reagent", that is optionally used to wash the cells, and thus needs to be bigger than the cell's pores, as will be explained in detail below.

Each of the steps of the method of the present invention is further explained below:
Step a) comprises or consists of adding to the sample reagents to perform an intracellular isothermal amplification of the target nucleic acid molecule comprised in the first population of cells and not in the second population of cells wherein the isothermal amplification uses a first molecule that is incorporated in the resulting amplified target nucleic acid molecules, thereby labelling said amplified target nucleic acid molecules.

The aim of this step, also called herein the "amplification and labelling step", is to selectively amplify and label the target nucleic acid molecule that is present only in the first population of cells. Thus, although the reagents necessary for said amplification are added to the sample and thus will be in contact with the at least the two population of cells comprised in the sample, the amplification must be specific for the target nucleic acid molecule, which is only present in the first population of cells. This way, the isothermal amplification only occurs in the first population of cells, and not in the second population of cells, and the amplified target nucleic acid molecules will be present only in the first population of cells.

The isothermal amplification of step a) occurs inside the cell (also referred herein as to in-cell isothermal amplification or intracellular isothermal amplification), so that the amplified target nucleic acid molecules (also called herein "the amplicons") remain inside the cells of the first population of cells. Isothermal amplification is a molecular biology technique known by the skilled person that is used for the amplification of DNA or RNA at a constant temperature, eliminating the need for the thermal cycling equipment utilized in traditional PCR (polymerase chain reaction). Any isothermal amplification methods are suitable to be used in this step. Preferably, the isothermal amplification method is selected from the list consisting of Loop-Mediated Isothermal Amplification (LAMP), Whole Genome Amplification (WGA), Strand Displacement Amplification (SDA), Helicase-Dependent Amplification (HDA), Recombinase Polymerase Amplification (RPA), Nucleic Acid Sequences Based Amplification (NASBA), Rolling circle amplification (RCA), Hyperbranched Rolling Circle Amplification (HRCA).

Preferably, the in-cell isothermal amplification method used in step b) is LAMP.

Components of an amplification reaction may include, but are not limited to, e.g., primers (e.g., individual primers, primer pairs, primer sets and the like) a polynucleotide template, polymerase, nucleotides, dNTPs and the like. In some embodiments, non-naturally occurring nucleotides or nucleotide analogues, such as analogues containing a detectable label (e.g., fluorescent or colorimetric label), may be used for example. Polymerases can be selected by a person of ordinary skill and include polymerases for thermocycle amplification (e.g., Taq DNA Polymerase; Q-Bio^{™} Taq DNA Polymerase (recombinant truncated form of Taq DNA Polymerase lacking 5'-3'exo activity); and polymerases for thermostable amplification (e.g., RNA polymerase for transcription-mediated amplification (TMA). Other enzyme components can be added, such as reverse transcriptase for transcription mediated amplification (TMA) reactions, for example.

The isothermal amplification is performed using a first molecule that is used to label the amplified nucleic acid molecules. The term "label" denotes any molecule that can be detectable or isolatable, and that is capable to bind to the second molecule as defined in the present invention. Examples of labels include, but are not limited to, a fluorescent label, a radioactive label, a paramagnetic particle, a chemiluminescent label, a label that is detectable by virtue of a secondary enzymatic reaction, and a label that is detectable by virtue of binding to a molecule (such as biotin). Thus, when the amplified target nucleic acid becomes labelled, it becomes detectable.

This first molecule may be any reagent or molecule as long as it is able to bind to the newly synthesized nucleic acid (i.e., the amplicons), thereby resulting in labelled amplified target nucleic acid molecules or amplicons. The amplicons include naturally occurring nucleotides, non-naturally occurring nucleotides, nucleotide analogs and the like and combinations of the foregoing. An amplification product often has a nucleotide sequence that is identical to or substantially identical to a nucleic acid sequence herein, or complement thereof. A "substantially identical" nucleotide sequence in an amplification product will generally have a high degree of sequence identity to the nucleotide sequence species being amplified or complement thereof (e.g., about 75%, 76%, 77%, 78%, 79%, 80%, 81 %, 82%, 83%, 84%, 85%, 86%, 87%, 88%, 89%, 90%, 91 %, 92%, 93%, 94%, 95%, 96%, 97%, 98%, 99% or greater than 99% sequence identity), and variations sometimes are a result of infidelity of the polymerase used for extension and/or amplification, or additional nucleotide sequence(s) added to the primers used for amplification

In an embodiment, the first molecule used in step a) is bound to the dNTPs (dUTPs, dATPs, dCTPs, dGTPs, dTTPs) used in the in-cell isothermal amplification, preferably bound to the dUTPs, so that the amplified nucleic acids are labelled in their nucleobases. Preferably, the first molecule is biotin. Preferably, said biotin is bound to the dNTPs (dUTPs, dATPs, dCTPs, dGTPs, dTTPs) used in the in-cell isothermal amplification, preferably bound to the dUTPs.

In an embodiment, the first molecule used in step a) is a molecule that is bound to the primers used for the intracellular isothermal amplification, preferably LAMP. In this case, the amplicons will comprise the first molecule in their 5' region that correspond to the primer used for said amplification.

The first molecule can thus be any molecule suitable to be incorporated or attached to the amplified nucleic acid, thereby labelling it. In an embodiment, the first molecule is selected from the list consisting of biotin, magnetic particles, fluorescent moieties, Fluorophores (SYBR Green, Etidio Bromuro (EtBr), DAPI, Fluoresceine, Alexa Fluor), Radioisotopes ([^32P], [^35S] ); bioluminiscents molecules (Digoxigenina, Biotin, Luminol), enzymes (HRP); others (PNA, LNA) etc.

In an embodiment, the first molecule used in step a) is a fluorescent label, such as FAM. This fluorescent label may be incorporated in the amplified target nucleic acid by using labelled primers, such as primer-FAM.

In an embodiment, the first molecule used in step a) is a magnetic label, such as gold nanoparticles (AuNPs). This magnetic label may be incorporated in the amplified target nucleic acid by using primers bound to said magnetic label, such as primer-AuNPs.

In an embodiment, before carrying out the intracellular isothermal amplification, the cells are treated with reagent to favour the entry of the intracellular isothermal amplification reagents into the cells. In an embodiment, the cells are permeabilized and/or fixed. Thus, this step corresponds to standard fixating and permeabilizing methods known in the art.

In an embodiment, the fixation reagent is selected from the group consisting of ACME, Acetone+Methanol, Acetic Acid + methanol, Formaldehyde, parafolmaldehyde, methanol, ethanol, acetic acid + etanol.

In an embodiment, the permeabilization reagent is selected from the group consisting of Triton X-100 , Saponina, Digitonina, Tween 20, NP-40, EDTA, Zwittergentes (CHAPS, CHAPSO).

Preferably, the reagents used in this step are both a fixation and a permeabilization reagent. Preferably, when the target nucleic acid molecule is a RNA molecule, the reagent used in step a) is ACME.

As a result of the permeabilization and fixation step, the sample is fixed and permeabilized, so that the first and second population of cells are fixed and permeabilized and are ready to proceed with the isothermal amplification of step a).

Another way of preparing the cells for the intracellular isothermal amplification of step a) may be incubating the sample with liposomes that carry the reagents of the intracellular isothermal amplification. This way, the liposomes act as a delivery vehicle to deliver said reagents inside the cells.

A further way of preparing the cells for the intracellular isothermal amplification of step a) may be subjecting them to electroporation, so that pores are created in their membrane that allow the entry of the reagents needed for the intracellular isothermal amplification.

If the target nucleic acid molecule is a RNA molecule, a step to convert the RNA into DNA is performed before proceeding with the intracellular isothermal amplification. Preferably, the target nucleic acid molecule is a RNA molecule, and the intracellular isothermal method is reverse transcription LAMP (RT- LAMP) detection.

Thus, when the target nucleic acid molecule is a RNA molecule, such as a mRNA molecule, step
a) comprises the sub-steps of (RT- LAMP):
   a.1) adding to the sample reagents to perform an intracellular reverse transcription of the target RNA molecule to provide a reverse transcribed DNA nucleic acid, and
   a.2) adding to the sample reagents to perform an intracellular isothermal amplification of the product of step a.1) (i.e. the reverse transcribed DNA nucleic acid) comprised in the first population of cells and not in the second population of cells, wherein the isothermal amplification uses a first molecule that is incorporated in the resulting reverse transcribed amplified DNA nucleic acid, thereby labelling said reverse transcribed amplified DNA nucleic acid.

As a result of step a), the first population of cells will comprise amplified target nucleic acid molecules, namely DNA amplicons, that are labelled with the first molecule (also referred herein as to labelled amplicons or labelled amplified target nucleic acid molecules). However, the second population of cells will not have labelled amplicons since the amplification did not occur in said cells as they lacked the target nucleic acid molecule.

In an embodiment, one or more washing steps can be performed between step a) and b).

Step b) comprises or consists of detecting and separating the first population of cells from the second population of cells using a separation method that detects the presence of the labelled amplified target nucleic acid molecules in the first population of cells and separates the first population of cells from the second population of cells in virtue of the presence of said labelled amplified target nucleic acid molecules.

The aim of step b) is to separate the first population of cells from the second population of cells, based on the presence of the labelled amplicons only in the first population of cells. Thus, this step is a detection and separation step.

In an embodiment, the first molecule used in step a) is a fluorescent label, and step b) comprises detecting and separating the cells of the first population of cells by cell sorting based on the presence of fluorescently labelled amplicons. This can be achieved for example by using in the intracellular isothermal amplification of step a) a set of primers labelled with fluorescent moieties (the first molecule), and cell sorting them by FACs. An example of a fluorescent moiety is FAM. An example of this embodiment is represented in Fig. 2.

Thus, in an embodiment, the method comprises the steps of:
a) adding to the sample reagents to perform an intracellular isothermal amplification, preferably LAMP, of the target nucleic acid molecule comprised in the first population of cells and not in the second population of cells wherein the isothermal amplification uses a fluorescent moiety that is incorporated in the resulting amplified target nucleic acid molecules, thereby labelling said amplified target nucleic acid molecules, and
b) detecting and separating the first population of cells from the second population of cells using a separation method that detects the presence of the fluorescent labelled amplified target nucleic acid molecules in the first population of cells and separates the first population of cells from the second population of cells in virtue of the presence of said fluorescent labelled amplified target nucleic acid molecules.

In an embodiment, the first molecule used in step a) is a magnetic label, and step b) comprises detecting and separating the cells of the first population of cells using a magnetic field, such as a magnet. This can be achieved for example by using in the intracellular isothermal amplification of step a) a set of primers labelled with gold nanoparticles (the first molecule).

Thus, in another embodiment, the method comprises the steps of:
a) adding to the sample reagents to perform an intracellular isothermal amplification, preferably LAMP, of the target nucleic acid molecule comprised in the first population of cells and not in the second population of cells wherein the isothermal amplification uses a magnetic moiety that is incorporated in the resulting amplified target nucleic acid molecules, thereby magnetically labelling said amplified target nucleic acid molecules, and
b) detecting and separating the first population of cells from the second population of cells using a magnetic field.

Washing steps to remove the excess of the first molecule are also contemplated in the present invention, preferably between steps a) and b).

More sophisticated forms of performing step b) are also contemplated. In a preferred embodiment, step b) comprises the sub-steps of:
b.i) incubating the cells comprised in the sample with a first reagent that comprises a second molecule that binds to the first molecule used in step a), thereby forming labelled amplified amplicons-first reagent complexes inside the cells of the first population of cells,
b.ii) washing the unbound first reagent, and
b.iii) subjecting the cells comprised in the sample to a separation method that detects the presence of the labelled amplified amplicons-first reagent complexes in the first population of cells and separates them.

Step b.i) comprises or consists of adding a first reagent to the sample (i.e., to the first and second population of cells), wherein the first reagent comprises a second molecule that binds to the first molecule, thereby forming a complex with it. Since the first molecule is bound to the labelled amplified target nucleic acid molecules (or labelled amplicons), once the second molecule is contacted with the first molecule, labelled amplified amplicons-first reagent complexes will be formed inside the cells of the first population of cells but not in the second population of cells.

The second molecule can be any molecule as long as it is capable of entering in the cell and bind and form a complex with the first molecule. For example, if the first molecule is biotin, the second molecule may be streptavidin.

The first reagent used in step b.i) can be any reagent or molecule or compound as long as it can be functionalized with a second molecule, and as long as it has a size that allows it to penetrate in the cells of the first population of cells, so that the second molecule can bind to the first molecule.

Preferably, the first reagent used in step b.i) comprises a targeting moiety, preferably an antibody. Said antibody will bind to the first molecule, and form labelled amplified amplicons-antibody complexes that will be subsequently separated, thereby separating the first population of cells from the second population of cells.

Preferably, the first reagent used in step b.i) is a population of magnetic nanoparticles (MNPs) that have a size smaller than the cell's pores, so that they can enter in the cell. Preferably, the MNPs have a size of less than 50nm. Preferably, the MNPs have diameter of between 10-50 nm, more preferably 10-20 nm, most preferably 10 nm.

Thus, in this preferred embodiment, step b.i) comprises incubating the sample with a population of MNPs, wherein each MNPs is coated or bound to a second molecule, wherein the second molecule binds to the first molecule, thereby forming labelled amplified amplicons-MNPs complexes that will be only present inside the first population of cells.

Once the labelled amplified amplicon-first reagent complexes are formed, step b.ii) is performed to wash the unbound first reagent and to prepare the sample for step b.iii).

Washing steps are standard and known in the art. However, in a preferred embodiment, the washing step b.ii) is performed by incubating the sample with a second reagent that is unable to penetrate in the cells of the first population of cells, and that is functionalized with a third molecule that binds to the second molecule used in step b.i). The aim of this washing step is to attract the unbound second molecule outside the cell of the first population of cells, thereby washing the excess of the first reagent. The second reagent used in step b.ii) can be any reagent or molecule or compound as long as it can be functionalized with a third molecule, and as long as it has a size that does not allow said reagent to penetrate in the cells of the first population of cells, so that the second molecule needs to leave the cell to bind to the third molecule functionalized in the second reagent.

Preferably, the second reagent is a population of microparticles (MPs), so that the washing step b.ii) is performed by incubating the sample with population of MPs that are unable to penetrate in the cells of the first population of cells, wherein each MP is functionalized with a third molecule that binds to the second molecule used in step b.i). An example of this method is represented in Fig. 3, Step 2C using MNPs_Streptavidin that are washed in step 3 with Reagent 2 that are MPs having Biotin (molecule 3) that binds to biotin (molecule 2).

The third molecule can be any compound or molecule as long as it is able to bind the second molecule. Preferably, the first molecule used in step a), and the third molecule used in step b.iii), if present, is biotin, and the second molecule used in step b.i), if present, is streptavidin.

Preferably:
- the first molecule used in step a) is biotin,
- the first reagent in step b.i) is a population of MNPs, wherein each MNP is functionalized with streptavidin, and
- the second reagent in step b.ii) is a population of MPs, wherein each MP is functionalized with biotin.

As stated above, the second reagent, preferably a population of MPs, has a size that is bigger than the cellular membrane pores, so that it is not able to move inside the cells. Preferably, the second reagents is a population of MPs that have a size bigger than 50 nm. More preferably, the MPs have a diameter of at least 50 nm, more preferably between 6-8 µm, most preferably 6.8 µm. Preferably, the MPs are polystyrene microparticles.

The embodiment of using MPs in step b.ii) is preferably combined with the embodiment of using MNPs in step b.i). Thus, it is a preferred embodiment that step b) comprises the following sub-steps:
b.i) incubating the cells comprised in the sample with a population of MNPs, wherein each MNP comprises or is functionalized with a second molecule that binds to the first molecule used in step a), thereby forming labelled amplified amplicons-MNPs complexes inside the cells of the first population of cells,
b.ii) washing the unbound first reagent by incubating the sample with a population of MPs, wherein each MPs comprises or is functionalized with a third molecule that binds to the second molecule used in step b.i), and
b.iii) subjecting the cells comprised in the sample to a detection and separation method that detects the presence of the labelled amplified amplicons-MNPs complexes in the first population of cells and separates them.

Step b.iii) comprises or consists of separating the labelled amplified amplicons-first reagent complexes that are only formed inside the first population of cells, thereby separating the first population of cells from the second population of cells.

In an embodiment, when step b.i) was performed using MNPs, step b.iii) preferably comprises subjecting the cells comprised in the sample to a magnetic field, so that the first population of cells are attracted to the magnetic field due to the presence of labelled amplified nucleic acid-MNPs complexes. Preferably, the magnetic field is applied using a magnet.

Thus, it is a preferred embodiment that step b) comprises the following sub-steps:
b.i) incubating the cells comprised in the sample with a population of MNPs, wherein each MNP comprises or is functionalized with a second molecule that binds to the first molecule used in step a), thereby forming labelled amplified amplicons-MNPs complexes inside the cells of the first population of cells,
b.ii) washing the unbound reagent by incubating the sample with a population of MPs, wherein each MPs is functionalized with a third molecule that binds to the second molecule used in step b.i), and
b.iii) subjecting the cells comprised in the sample to a magnetic field, thereby separating the first population of cells from the second population of cells.

Other separation methods can be used in step b.iii), such as using fluorescence moieties in the first or second reagents. For example, in an embodiment, step b.i) is performed using a population of MNPs, wherein each MNP comprises or is functionalized with a second molecule that binds to the first molecule used in step b) and wherein each MNP is labelled with a fluorescent moiety, and step b.iii) comprises separating the cells based on the presence of fluorescently labelled amplified nucleic acid-MNPs complexes. Other separation methods include Such as FACS (Fluorescence-Activated Cell Sorting), MACS (Magnetic-Activated Cell Sorting).

The following embodiments are preferred embodiments of the method of the first aspect:
In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that detects the presence of the labelled amplified target nucleic acid molecules in the first population of cells and separates the first population of cells from the second population of cells in virtue of the presence of said labelled amplified target nucleic acid molecules,
wherein the first molecule is a fluorescent moiety that is bound to the primers used in the intracellular amplification, and wherein step b) comprises detecting and separating the cells of the first population of cells by cell sorting based on the presence of fluorescently labelled amplicons.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that detects the presence of the labelled amplified target nucleic acid molecules in the first population of cells and separates the first population of cells from the second population of cells in virtue of the presence of said labelled amplified target nucleic acid molecules,
wherein the first molecule is a fluorescent moiety that is bound to the dNTPs used in the intracellular amplification, and wherein step b) comprises detecting and separating the cells of the first population from the cells of the second population of cells by cell sorting based on the presence of fluorescently labelled amplicons.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that detects the presence of the labelled amplified target nucleic acid molecules in the first population of cells and separates the first population of cells from the second population of cells in virtue of the presence of said labelled amplified target nucleic acid molecules,
wherein the first molecule is a magnetic moiety that is bound to the primers used in the intracellular amplification, and wherein step b) comprises detecting and separating the cells of the first population from the cells of the second population of cells by applying a magnetic field.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that detects the presence of the labelled amplified target nucleic acid molecules in the first population of cells and separates the first population of cells from the second population of cells in virtue of the presence of said labelled amplified target nucleic acid molecules,
wherein the first molecule is a magnetic moiety that is bound to the dNTPs used in the intracellular amplification, and wherein step b) comprises detecting and separating the cells of the first population from the cells of the second population of cells by applying a magnetic field.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
   b.i) incubating the cells comprised in the sample with a first reagent that comprises a second molecule that binds to the first molecule used in step b), thereby forming labelled amplified amplicons-first reagent complexes inside the cells of the first population of cells,
   b.ii) washing the unbound first reagent using a second reagent comprising a third molecule that binds to the second molecule used in step b.i), wherein the second reagent is bigger than the cell's membrane pores and thus it cannot enter in the cells of the first population of cells., and
   b.iii) subjecting the cells comprised in the sample to a separation method that detects the presence of the labelled amplified amplicons-first reagent complexes in the first population of cells and separates them.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
   b.i) incubating the cells comprised in the sample with a first reagent, preferably a population of MNPs, that comprises a second molecule that binds to the first molecule used in step b), thereby forming labelled amplified amplicons-first reagent complexes inside the cells of the first population of cells,
   b.ii) washing the unbound first reagent using a second reagent, preferably a population of MPs, comprising a third molecule that binds to the second molecule used in step b.i), wherein the second reagent is bigger than the cell's membrane pores and thus it cannot enter in the cells of the first population of cells., and
   b.iii) subjecting the cells comprised in the sample to a separation method that detects the presence of the labelled amplified amplicons-first reagent complexes in the first population of cells and separates them, wherein the separation method is preferably applying a magnetic field.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
   b.i) incubating the cells comprised in the sample with a first reagent that comprises a second molecule that binds to the first molecule used in step b), thereby forming labelled amplified amplicons-first reagent complexes inside the cells of the first population of cells,
   b.ii) washing the unbound first reagent, and
   b.iii) subjecting the cells comprised in the sample to a separation method that detects the presence of the labelled amplified amplicons-first reagent complexes in the first population of cells and separates them,
   wherein the first reagent is a population of MNPs, wherein each MNPs is coated or functionalized with a second molecule, and wherein step b.iii) is performed by subjecting the cells to a magnetic field.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
   b.i) incubating the cells comprised in the sample with a first reagent that comprises a second molecule that binds to the first molecule used in step b), thereby forming labelled amplified amplicons-first reagent complexes inside the cells of the first population of cells,
   b.ii) washing the unbound first reagent, and
   b.iii) subjecting the cells comprised in the sample to a separation method that detects the presence of the labelled amplified amplicons-first reagent complexes in the first population of cells and separates them,
wherein the first molecule is bound to the dNTPs used in the intracellular amplification, wherein the first reagent is a population of MNPs, wherein each MNP is coated or functionalized with a second molecule, and wherein step b.iii) is performed by subjecting the cells to a magnetic field.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
   b.i) incubating the cells comprised in the sample with a first reagent that comprises a second molecule that binds to the first molecule used in step b), thereby forming labelled amplified amplicons-first reagent complexes inside the cells of the first population of cells,
   b.ii) washing the unbound first reagent, and
   b.iii) subjecting the cells comprised in the sample to a separation method that detects the presence of the labelled amplified amplicons-first reagent complexes in the first population of cells and separates them,
wherein the first molecule is bound to the primers used in the intracellular amplification, wherein the first reagent is a population of MNPs, wherein each MNP is coated or functionalized with a second molecule, and wherein step b.iii) is performed by subjecting the cells to a magnetic field.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification, preferably LAMP, of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
   b.i) incubating the cells comprised in the sample with a population of MNPs, wherein each MNP comprises or is functionalized with a second molecule that binds to the first molecule used in step a), thereby forming labelled amplified amplicons-MNPs complexes inside the cells of the first population of cells,
   b.ii) washing the unbound reagent by incubating the sample with a population of MPs, wherein each MPs is functionalized with a third molecule that binds to the second molecule used in step b.i), and
   b.iii) subjecting the cells comprised in the sample to a magnetic field, thereby separating the first population of cells from the second population of cells,
   wherein the first molecule is bound to the primers or the dNTPs used in the intracellular amplification.

In an embodiment, the method of the first aspect is an *in vitro* method for separating a first population of cells from a second population of cells comprised in a sample, wherein the first population of cells is characterized in that they comprise at least one target nucleic acid molecule that is not present in the second population of cells, the method comprising the steps of:
a) adding to the sample reagents to perform an intracellular isothermal amplification of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
   b.i) incubating the cells comprised in the sample with a first reagent that comprises a second molecule that binds to the first molecule used in step b), thereby forming labelled amplified amplicons-first reagent complexes inside the cells of the first population of cells,
   b.ii) washing the unbound first reagent, and
   b.iii) subjecting the cells comprised in the sample to a separation method that detects the presence of the labelled amplified amplicons-first reagent complexes in the first population of cells and separates them,
wherein the first molecule is bound to the primers or to the dNTPs used in the intracellular amplification, wherein the first reagent is a population of MNPs, wherein each MNP is coated or functionalized with a second molecule and a fluorescent moiety, and wherein step b.iii) is performed by subjecting the cells cell sorting based on the presence of the fluorescent moiety.

In an embodiment, the method is implemented by a device or kit, and wherein said device or kit comprises:
a) means and reagents to perform an intracellular isothermal amplification of the target nucleic acid molecule, and
b) means and reagents to separate the first population of cells from the second population of cells using a separation method that detects the presence of the labelled target nucleic acid molecule in the first population of cells.

Preferably, the device or kit also comprises means and reagents to prepare the cells for step a), such as means and reagents fixate and permeabilize the first and the second population of cells.

In **a second aspect,** the present invention provides an *in vitro* method to identify if a population of cells carrying genetic mutations or variants, the method comprising the steps of:
i. carrying out the method as defined in the first aspect of the present invention, or any of its embodiments, thereby separating the first population of cells from the second population of cells, and
ii. sequencing the genome, or a part thereof, of the first population of cells isolated in step i. and detecting the presence of said genetic disease or variants.

Thus, this second aspect is aimed at detecting or identifying genetic mutations or variants that may be present in the first population of cells. Step i. provides the separation and enrichment of the first population of cells, so that step ii. allows the sequencing of the genome of said cells without contamination from the second population of cells.

Step ii. can be performed with any sequencing methods known in the art. Sequence reads can be mapped and the number of reads or sequence tags mapping to a specified nucleic acid region (e.g., a chromosome, a bin, a genomic section) are referred to as counts. In some embodiments, counts can be manipulated or transformed (e.g., normalized, combined, added, filtered, selected, averaged, derived as a mean, the like, or a combination thereof). In some embodiments, counts can be transformed to produce normalized counts. The sequence reads can be analysed using a computer implemented method any apparatus suitable for said purposes. Thus, the method of the second aspect can be implemented in apparatus for high-throughput sequencing.

The genetic mutations or variants that are identified or detected in step ii. may include allelic variants, ratio of the alleles, gene copy numbers, deletions, a genetic duplications, an insertions, a mutations, genetic aberration, polymorphisms, SNPs, Chromosomal anomalies, microdelections, autosomal recessive mutations, x-linked mutations, autosomal dominant monogenic diseases, etc.

The term "gene copy number" as used herein refers to the amount or quantity of total nucleic acid or fetal nucleic acid. The method of the second aspect thus provides a process for determining the absolute amount of fetal nucleic acid in a mixed sample comprising cells from the mother and the foetus. Absolute amount or copy number represents the number of molecules available for detection and may be expressed as the genomic equivalents per unit.

The terms "ratio of the alleles" or "allelic ratio" as used herein refer to the ratio of the population of one allele and the population of the other allele in a sample. In some trisomic cases, it is possible that a fetus may be tri-allelic for a particular locus. In such cases, the term "ratio of the alleles" refers to the ratio of the population of any one allele against one of the other alleles, or any one allele against the other two alleles. The method of the second aspect would allow to identify different ratio of the alleles between the first population of cells (fetus cells) and the second population of cells (mother's cells).

The terms "single nucleotide polymorphism" or "SNP" refer to the polynucleotide sequence variation present at a single nucleotide residue within different alleles of the same genomic sequence. This variation may occur within the coding region or non-coding region (i.e., in the promoter or intronic region) of a genomic sequence, if the genomic sequence is transcribed during protein production. Detection of one or more SNP allows differentiation of different alleles of a single genomic sequence or between two or more individuals, such as the foetus and their mother. The term "allele" as used herein is one of several alternate forms of a gene or non-coding regions of DNA that occupy the same position on a chromosome. The term allele can be used to describe DNA from any organism including but not limited to bacteria, viruses, fungi, protozoa, molds, yeasts, plants, humans, non-humans, animals, and archeabacteria.

Provided herein are methods, processes and apparatuses useful for identifying a genetic variation in a population of cells. In some embodiments identifying a genetic variation comprises detecting a copy number variation and/or sometimes comprises detecting SNPs, mutations, insertions, deletions, etc. In some embodiments, identifying a genetic variation by a method described herein can lead to a diagnosis of, or determining a predisposition to, a particular medical condition. Identifying a genetic variance can result in facilitating a medical decision and/or employing a helpful medical procedure. The term "a genetic variation", as referred to herein, means one or more genetic variations.

The method of the second aspect may therefore be used for foetal diagnosis when the method of the first aspect is performed in an isolated maternal biological sample (such as maternal whole blood sample) having cells from the mother and from the foetus.

In some embodiments, presence or absence of a genetic variation (e.g., chromosome aneuploidy e.g., full chromosome aneuploidy, partial chromosome aneuploidy or segmental chromosomal aberration (e.g., mosaicism, deletion and/or insertion)) is determined for a foetus. In such embodiments, presence or absence of a foetal genetic variation (e.g., foetal chromosome aneuploidy) is determined. In certain embodiments, presence or absence of a genetic variation (e.g., chromosome aneuploidy) is determined for a sample. In such embodiments, a variation detected resides in the first population of cells (preferably foetal cells) but not in the second population of cells (preferably maternal cells).

The method of the second aspect may also be used to identify or detect cell pathological conditions in a heterogeneous sample, as well as classifying microorganisms based on their genetic variations.

The method of the second aspect may also be a method for analyse the genotype of the foetus, the method comprising the steps of:
i. enriching foetal cells from a maternal sample, preferably blood sample, by performing the method of the first aspect or any of its embodiments, and
ii. sequencing the genome, or part thereof, of said enriched foetal cells.

The genotype of foetal cells can be analysed for any purpose. Typically, the genotype will be analysed to detect the likelihood that the offspring will possess a trait of interest or a genetic variant or abnormality. Preferably, the foetal cells are analysed for a genetic abnormality linked to a disease state, or predisposition thereto. In one embodiment, the genetic abnormality is in the structure and/or number or chromosomes. In another embodiment, the genetic abnormality encodes an abnormal protein. In another embodiment, the genetic abnormality results in decreased or increased expression levels of a gene.

In a third aspect, the present invention provides a computer implemented method for carrying out the method of the first or second aspects.

In a **fourth aspect,** the present invention provides a device or kit or system comprising means and reagents to carry out the method of the first and/or of the second aspect, or any of their embodiments.

Each embodiment disclosed herein is contemplated as being applicable to each of the other disclosed embodiments. Thus, all combinations of the various elements described herein are within the scope of the invention.

### EXAMPLES

**EXAMPLE 1. Example of the general method:** This example is represented in Fig. 1 and shows the whole process of separation of a specific cell population form a mix of cell populations. On the left, the different steps that occur in the tube are represented. Briefly, there is a mix of cells comprising at least a first and a second population of cells, which are differentiated in that in the first population of cells there is a target nucleic acid (NA) that is not present in the second population of cells. The first population of cells is then subjected to a targeted intracellular NA isothermal amplification where the amplicons are labelled with a first molecule (also called Molecule 1), and then the cells of the first population are separated based on the presence of these labelled amplicons. On the right of Fig. 1, an amplified image of the isothermal amplification and NA labelling process is shown. Step 1 comprises amplifying the target NA where the amplicons are labelled with Molecule 1. Then, in Step 2, Reagent 1 (the first reagent) is added to the mix. Reagent 1 contains Molecule 2 (the second molecule), which targets Molecule 1. After that, Reagent 2 can be washed out (Step 3) with Reagent 2, which contains Molecule 3 that targets Molecule 2. This process results in the efficient separation of the cell population having the target NA labelled with Molecule 1.

**EXAMPLE 2: Separation of fetal erythroblasts from other blood populations using primers labelled with FAM.** This example is represented in Fig. 2 and shows an experiment where fetal erythroblasts were separated from blood based on the intracellular amplification of fetal hemoglobin (HbF). As explained before, the process consists in amplifying a target nucleic acid, in this case the HbF mRNA, which is specific of fetal erythroblasts, see Fig. 2a. We used two different samples: blood from a non-pregnant woman (as a negative control because their blood does not contain fetal erythroblasts, but it does contain adult erythroblasts) and umbilical cord blood (where all the erythroblasts should express HbF). Red blood cells were lysed, obtaining the peripheral blood mononuclear cells (PBMCs), which were fixed and permeabilized, and the intracellular isothermal amplification was performed. In this experiment, we used Reverse transcription-loop-mediated isothermal amplification (RT-LAMP), as we wanted to amplify and label an mRNA. We used 6 primers for LAMP and one of them, FIP were bound to FAM. Therefore, when amplifying the HbF, the DNA is labeled with FAM. FAM is thus a first molecule (Molecule 1) according to the present invention. The RT-LAMP reaction was performed for 1h at 65°C, were the fetal cells in suspension containing the HbF were labelled with FAM. Then, the cells were analyzed in the cytometer. The PBMCs form the non-pregnant women were negative for FAM while some fetal cells from the umbilical cord blood were positive for FAM, indicating that they were fetal erythroblasts, as they expressed HbF. Later, the fetal cells were sorted by FACS, see Fig. 2B.

In this example, since the amplicons were fluorescently labelled, Steps 2 and 3 were not needed to separate the target cells.

**EXAMPLE 3: Separation of fetal erythroblasts from other umbilical cord blood populations using dUTPs labelled with Biotin.** This example corresponds to Fig. 3.As explained in the previous example, this example shows an experiment where fetal erythroblasts were separated from blood based on the intracellular amplification of fetal hemoglobin (HbF). However, in this case Molecule 1 corresponds to Biotin that is bound to the dUTPs used. Steps 2 and 3 were carried out, see fig 3A. We used umbilical cord blood. Red blood cells were lysed, obtaining PBMCs, which were fixed and permeabilized, and RT-LAMP was performed. We used the same 6 primers as before, but this time, FIP primers were not labelled with fluorescence. In this case, Molecule 1 was Biotin bound to dUTPs. RT-LAMP reaction was performed as before. After the amplification, cells were washed and incubated with:

**Step 2B in** **Fig. 3A****:** 3 samples were used: one blank, one sample where the LAMP reaction was performed without the polymerase as a negative control, and one sample where the LAMP reaction was performed. In this case, Molecule 2 were antibodies anti-Biotin bound to FITC. Cells were incubated for 1h at RT and washed and analyzed by cytometry. As it can be observed, only the third sample (dUTPs_Biotin_FITC) shows positive signal for FITC, indicating that the in-cell RT-LAMP worked.

**Step 2C in** **Fig. 3A****:** As in Step 2B, 3 samples were used; one blank, one sample where the LAMP reaction was performed without the polymerase as a negative control, and one sample where the RT-LAMP reaction was performed. In this case, Reagent 1 were magnetic nanoparticles (MNPs) and Molecule 2 was Streptavidin (against Biotin) and cyanin_5 (Cy5). Cells were incubated for 1 h at RT and washed. In addition, remaining not bound MNPs_Strpt_Cy5 were washed out using a second Reagent (microparticles (MPs)) bound to Biotin (Molecule 3), see step 3C in Fig. 3A. Cells were washed for 1h at RT and then analyzed by cytometry, see Figs. 3B and 3C. As it can be observed, the third sample (dUTPs_Biotin_MNPs_Cy5) was positive for Cy5, indicating that Molecule 2 was bound to Molecule 1. It is worth mentioning that in this case, cells could have been isolated by a magnet, as they were charged with the MNPs. However, with the purpose of illustrating comprehensively the results, as they were also labeled with Cy5, they were isolated by FACS.

**EXAMPLE 4: Separation of fetal erythroblasts from other umbilical cord blood populations using primers labelled with Biotin.** This example is represented in Fig. 4, and shows the same process as Example 3 but instead of using Biotin (Molecule 1) bound to dUTPs, it is bound to primers, see Fig 4A. As it can be observed in Fig 4B and C, when using Molecule 1 bound to primers, the process also works. However, there is higher background in the sample where No Polymerase was added in both cases (B and C). This is due to the fact that primers are bigger than the dUTPs and more washes are required to eliminate their excess in the sample.

## Claims

1. An *in vitro* method for separating a first population of cells from a second population of cells comprised in a sample, wherein the first population of cells is **characterized in that** they comprise at least one target nucleic acid molecule that is not present in the second population of cells, the method comprising the steps of:
a) adding to the sample reagents to perform an intracellular isothermal amplification of the target nucleic acid molecule comprised in the first population of cells but not comprised in the second population of cells, wherein the isothermal amplification uses at least a first molecule that is incorporated in the resulting amplicons, thereby labelling said amplicons, and
b) detecting and separating the first population of cells from the second population of cells using a separation method that comprises the steps of:
b.i) incubating the cells comprised in the sample with a first reagent that comprises a second molecule that binds to the first molecule used in step b), thereby forming labelled amplified amplicons-first reagent complexes inside the cells of the first population of cells,
b.ii) washing the unbound first reagent, and
b.iii) subjecting the cells comprised in the sample to a separation method that detects the presence of the labelled amplified amplicons-first reagent complexes in the first population of cells and separates them.

2. The *in vitro* method according to claim 1, wherein step b.ii) is performed using a second reagent comprising a third molecule that binds to the second molecule used in step b.i), wherein the second reagent is bigger than the cell's membrane pores and thus it cannot enter in the cells of the first population of cells.

3. The *in vitro* method of any one of claims 1 or 2, wherein the first reagent used in step b.i) is a population of magnetic nanoparticles (MNPs), preferably MNPs with a diameter of between 10-50 nm, more preferably 10-20 nm.

4. The *in vitro* method of claim 3, wherein step b.iii) is performed by subjecting the cells comprised in the sample to a magnetic field.

5. The *in vitro* method of any of claims 2 to 4, wherein the second reagent is a population of microparticles (MPs), preferably wherein the MPs have a diameter of at least 50 nm, more preferably between 6-8 µm.

6. The *in vitro* method of claims 1 or 2, wherein the first reagent used in step b.i) is a targeting moiety, preferably an antibody.

7. The *in vitro* method according to any one of claims 1 to 6, wherein the first molecule used in step a) is bound to the dNTPs used in the intracellular amplification, preferably bound to the dUTPs.

8. The *in vitro* method according to any one of claims 1 to 6, wherein the first molecule used in step a) is bound to the primers used in the in-cell isothermal amplification.

9. The *in vitro* method of any one of claims 1 to 8, wherein the intracellular isothermal amplification of step a) is LAMP.

10. The *in vitro* method of any one of claims 1 to 9, wherein the first population of cells are foetal cells, and the second population of cells are cells from a pregnant woman carrying the foetus, and wherein the sample is a biological sample obtained from said pregnant woman, preferably maternal whole blood sample, more preferably maternal PBMCs.

11. The *in vitro* method according to claim 10, wherein the target nucleotide sequence amplified in step a) is the mRNA of foetal haemoglobin.

12. The *in vitro* method according to any one of claims 1 to 11, wherein one or more washing step are performed between each of the steps a) and b).

13. An *in vitro* method to identify if a population of cells carrying genetic mutations or variants, cells characteristic of any pathological condition as well as microorganisms based on taxonomy, the method comprising the steps of:
i. carrying out the method as defined in any one of claims 1 to 12, thereby separating the first population of cells from the second population of cells, and
ii. sequencing the genome, or a part thereof, of the first population of cells isolated in step i. and detecting the presence of the genetic disease or variants.

14. A device or kit or system comprising means and reagents to carry out the method of any one of claims 1 to 13.

15. A computer-implemented method for carrying out the methods as defined in any of claims 1 to 14.
